# EUROPÄISCHE PATENTSCHRIFT

(11) **EP 0 663 945 B1**
(45) Veröffentlichungstag und Bekanntmachung des Hinweises auf die Patenterteilung: **05.02.1997**
(21) Anmeldenummer: 93920844.3
(22) Anmeldetag: 30.09.1993
(51) Int. Cl.: C11D 3/48, A01N 37/20

(54) **REINIGENDES DESINFEKTIONSMITTEL**
CLEANSING DISINFECTANT
AGENT DE DESINFECTION A ACTION NETTOYANTE

(30) Priorität: 09.10.1992 DE 4234070
(43) Veröffentlichungstag der Anmeldung: 26.07.1995
(73) Patentinhaber: Henkel-Ecolab GmbH & Co. OHG, 40554 Düsseldorf (DE)
(72) Erfinder: HACHMANN, Klaus, D-40723 Hilden (DE); FRIESE, Carsten, D-22559 Hamburg (DE)
(74) Vertreter: Wilk, Hans-Christof, Dr.
(86) Internationale Anmeldenummer: EP9302670
(87) Internationale Veröffentlichungsnummer: WO9409105

(56) Entgegenhaltungen:
- EP-A- 0 156 275
- WO-A-91/13965
- DE-A- 4 005 784
- DATABASE WPI Derwent Publications Ltd., London, GB; AN 90-361964 & CA,A,2 012 288 (STERLING DRUG INC) 16. September 1990
- DATABASE WPI Derwent Publications Ltd., London, GB; AN 90-361964 & CA,A,2 012 288

## Beschreibung

Die Erfindung betrifft ein reinigendes wäßriges Desinfektionsmittel auf Basis von Umsetzungsprodukten aus
a) N-substituierten Propylendiaminen der Formel I:

   R₁-NH-CH₂-CH₂-CH₂-NH₂, (I)

   in der R₁ für einen linearen Alkylrest mit 12 bis 14 Kohlenstoffatomen steht, und
b) Verbindungen der Formel II:

   R₂-O-CO-CH₂-CH₂-CH(NH₂)-COOH, (II)

   in der R₂ ein Wasserstoffatom oder einen Alkylrest mit 1 bis 4 Kohlenstoffatomen bedeutet, wobei das molare Verhältnis von a : b 1 : 1 bis 1 : 2 beträgt, (Komposition U) und/oder deren Salzen mit anorganischen oder organischen Säuren, gegebenenfalls zusammen mit nichtionischen und/oder Amphotensiden, Komplexbildnern und organischen Lösungsmitteln gemäß EP 156 275, das dadurch gekennzeichnet ist, daß es als Lösungsvermittler Benzylalkohol und/oder 2-Phenoxyethanol und/oder 1-Phenoxypropanol enthält.

Herstellung und Verwendung der Komposition U sind in EP 156 275 beschrieben. Aus der W091/13965 sind Wirkstoffkombinationen mit U bekannt. Es stellte sich jedoch heraus, daß die dort zitierten Lösungen in Wasser allein und in Verbindung mit üblichen Lösungsmitteln wie Alkoholen und Glykolethern, Ethanol, Propanol, Isopropanol oder Butyldiglykol (Diethylenglykolmonobutylether) bei Raumtemperatur nicht immer klar löslich sind und sich bei längerer Lagerung in Schichten trennen. Dies liegt an dem ungünstigen temperaturabhängigen Klar- und Trübungspunkt dieser Lösungen.

Es wurde nun gefunden, daß man zu klaren reinigenden Desinfektionsmitteln kommt, wenn man zusätzlich zu üblichen Lösungsmitteln als Lösungsvermittler für die Komposition U Benzylalkohol und/oder 2-Phenoxyethanol und/oder 1-Phenoxypropanol verwendet. Bevorzugt wird ein Gemisch Benzylalkohol und 1-Phenoxypropanol, besonders bevorzugt im Mengenverhältnis von etwa 1 : 1.

Die Menge der Lösungsvermittler im gesamten Mittel beträgt etwa 3 bis etwa 30, vorzugsweise etwa 7 bis etwa 15 Gew.-%. Das Gewichtsverhältnis von Lösungsvermittler zu U beträgt etwa 1,0 : 6,0 bis 1,0 : 0,05, vorzugsweise etwa 1 : 0,5 bis 1 : 0,07.

Durch Einsatz dieser Alkohole als Lösungsvermittler konnte der bisherige Klarpunkt der Desinfektionsmittellösungen in üblichen Lösungsmitteln auf Temperaturen weit unter 20 °C gesenkt werden. Sie blieben über einen beobachteten Zeitraum von etwa 6 Monaten lagerstabil.

Dieses vorteilhafte Ergebnis blieb auch bestehen, als den Lösungen noch nichtionische Tenside, Gerüststoffe sowie gegebenenfalls Duftstoffe und/oder Korrosionsinhibitoren zugesetzt wurden.

Die Komposition U wird in Mengen von etwa 5 bis 40 Gew.-%, vorzugsweise etwa 10 bis 25 Gew.-% eingesetzt.

Wenn neben der antimikrobiellen Wirkung eine zusätzliche Reinigungswirkung erwünscht ist, können die Mittel ein oder mehrere oberflächenaktive Stoffe, vorzugsweise aus den Gruppen der nichtionischen Tenside und der Amphotenside enthalten.

Als nichtionische Tenside kommen beispielsweise Alkylpolyglykoside mit vorzugsweise 8 bis 22 C-Atomen im Alkylrest, Umsetzungsprodukte von 4 bis 40, vorzugsweise 4 bis 20 Moläquivalenten Ethylenoxid (E0) und/oder Propylenoxid (P0) mit Fettalkoholen, Fettsäuren, Fettaminen, Fettsäureamiden oder Alkansulfonamiden, deren Fettalkylreste vorzugsweise jeweils 8 bis 22-C-Atome enthalten, und mit Alkylphenolen in Betracht. Auch die endgruppenverschlossenen Derivate derartiger Alkoxylierungsprodukte, vorzugsweise mit Endgruppen, die 2 bis 10-C-Atome enthalten, kommen in Frage. Von besonderem Interesse sind Anlagerungsprodukte von 5 bis 16 Mol Ethylenoxid am Kokos- oder Talgfettalkohole, an Oleylalkohol sowie an Mono-, Di- oder Trialkylphenole sowie an Monoalkylcyclohexanole mit 6 bis 14 Kohlenstoffatomen in den Alkylresten. Es kann vorteilhaft sein, mit den schaumärmsten Vertretern dieser Gruppe zu arbeiten.

Zu derartigen nichtionischen Tensiden gehören beispielsweise die Handelsprodukte Dehypon ^{(R)} LS 24, LS 36, LS 45, LS 54, LT 24, LT 104, OCP 502 (Lieferant Henkel), Dehydol ^{(R)} LT 30 (Lieferant Henkel), Lutensol ^{(R)} LF 224, LT 30 (Lieferant BASF), Triton ^{(R)} CF 54 und DF 12 (Lieferant Röhm & Haas).

Die Mengen der nichtionischen Tenside betragen etwa 3 bis 20, vorzugsweise etwa 5 bis 10 Gew.-%.

Zu den geeigneten Amphotensiden gehören Derivate tertiärer aliphatischer Amine oder quartärer aliphatischer Ammoniumverbindungen, deren aliphatische Reste geradkettig oder verzweigt sein können und von denen einer eine Carboxy-, Sulfo-, Phosphono-, Sulfato- oder Phosphato-Gruppe trägt. Beispiele für derartige Amphotenside sind Dimethyl-tetradecyl-glycin, Dimethyl-hexadecyl-glycin, Dimethyl-octadecyl-glycin, 3-(Dimthyl-dodecylammonio)-1-propansulfonat und die unter den Bezeichnungen Dehyton ^{(R)} AB, CB und G (Lieferant Henkel) vertriebenen Amphotenside. Ihre Mengen betragen etwa 0 bis 10, vorzugsweise etwa 2 bis 5 Gew.-%.

Um auch bei Verwendung der erfindungsgemäßen Reinigungsmittelkonzentrate mit hartem Wasser eine klare Anwendungslösung und ein gutes Korrosionsverhalten zu gewährleisten, können die erfindungsgemäßen Reinigungs- und Desinfektionsmittel Komplexbildner bzw. Korrosionsinhibitoren enthalten. Diese werden vorzugsweise aus den Gruppen der Phosphonsäuren, Aminocarbonsäuren und deren Salzen, insbesondere deren Alkylisalzen, ausgewählt. Zu diesen Komplexierungsmitteln gehören beispielsweise die Alkali-, vorzugsweise die Natriumsalze der Methandiphosphonsäure, Hydroxyethan-1,1-diphosphonsäure, 1-Aminoethan-1,1-diphosphonsäure, Amino-trimethylenphosphonsäure), Ethylendiamin-tetra(methylenphosphonsäure), Diethylentriamin-penta(methylenphosphonsäure), 2-Phosphonobutan-1,2,4-tricarbonsäure, Nitrilotriessigsäure (NTA), Ethylendiamintetraessigsäure und Hydroxyethyl-ethylendiamintriessigsäure. Bevorzugt wird NTA. Als reiner Korrosionsinhibitor kann auch 1,2,3-Benzotriazol eingesetzt werden. Derartige Komplexbildner bzw. Korrosionsinhibitoren sind in den erfindungsmäßen Mitteln vorzugsweise in Mengen nicht über 6 Gew.-%, insbesondere etwa 0,5 Gew.-% bis 3 Gew.-%, enthalten.

Darüber hinaus können die erfindungsgemäßen Reinigungs- und Desinfektionsmittelkonzentrate in solchen Mitteln übliche Zusätze, wie Farbstoffe oder Duftstoffe, enthalten. Derartige übliche Zusatzstoffe sind in den erfindungsgemäßen Mitteln bevorzugt in Mengen nicht über 1 Gew.-% vorhanden.

Zusätzlich können die erfindungsgemäßen Desinfektionsmittel wasserlösliche organische Lösungsmittel, vorzugsweise aus den Gruppen der Alkohole mit 1 bis 4 C-Atomen, der Glykole mit 2 bis 4 C-Atomen und der aus diesen ableitbaren Diglykole und Diglykolether, enthalten. Derartige Lösungsmittel sind beispielsweise Methanol, Ethanol, Propanol, Isopropanol, Tert.-Butanol, Ethylenglykol, Propylenglykol, Butylenglykol, Diethylenglykol, Dipropylenglykol, Diethylenglykolmonomethylether, Diethylenglykolmonoethylether, Diethylenglykolmonopropylether und Diethylenglykolmonobutylether. Organische Lösungsmittel können in Mengen von etwa 5 bis 35, vorzugsweise etwa 10 bis 20 Gew.-% eingesetzt werden. Lösungsmittel und Lösungsvermittler sind bevorzugt in Mengen nicht über 40 Gew.-%, insbesondere in Mengen von etwa 10 Gew.- bis etwa 30 Gew.-%, in den erfindungsgemäßen Mitteln enthalten.

Aus der DE 40 05 784 Al sind Desinfektionsmittelkonzentrate und deren Verwendung als Mykobakterizid und Viruzid bekannt, die im wesentlichen 10 bis 60 Gew.-% Phenoxyethanol oder ein Gemisch aus 2-Phenoxypropanol-1 und 1-Phenoxypropanol-2, 0,5 bis 50 Gew.-% einer Guadinium und/oder einer quaternären Ammoniumverbindung, 3 bis 25 Gew.-% eines nichtionischen Tensids und 0,1 bis 10 Gew.-% einer organischen stickstoffhaltigen Basis wie Tetrabis-(2-hydroxypropyl)N,N',N'-ethylendiamin enthalten. Hier sollen je nach der stickstoffhaltigen Base Wirksamkeitssteigerungen des Phenoxyalkanal/quaternäre-Ammoniumverbindungen-Gemisches eintreten. Derartige Beobachtungen spielen im Falle der vorliegenden Erfindung keine Rolle. Dagegen wird über Löslichkeiten und deren Verbesserungsmöglichkeiten durch Benzylalkohol und/oder Phenoxyalkanole im Stand der Technik nichts ausgesagt.

### Beispiele

Mit folgender Zusammensetzung:
- 25 Gew.-%: Komposition U
- 5 - 10 Gew.-%: Lösungsmittel
- 0 - 10 Gew.-%: Lösungsvermittler
- 7 Gew.-%: C₁₂-C₁₄-Alkyl-9E0-butylether (Dehypon ^{(R)} LS 104 L)
- 0,5 Gew.-%: 1,2,3-Benzotriazol (Preventol ^{(R)} C8-100)
- 0,2 Gew.-%: NTA
Rest auf 100 Gew.-% Wasser
wurden verschiedene Konfektionierungen hergestellt und jeweils 100 ml auf eine Temperatur von etwa 0 °C abgekühlt. Alle Proben waren trübe. Dann wurde langsam erwärmt und das Eintreten des Klarpunkts in °C bestimmt. Die Ergebnisse sind der nachfolgenden Tabelle zu entnehmen und zeigen die Vorteile der erfindungsgemäßen Lösungsvermittlerzusätze.

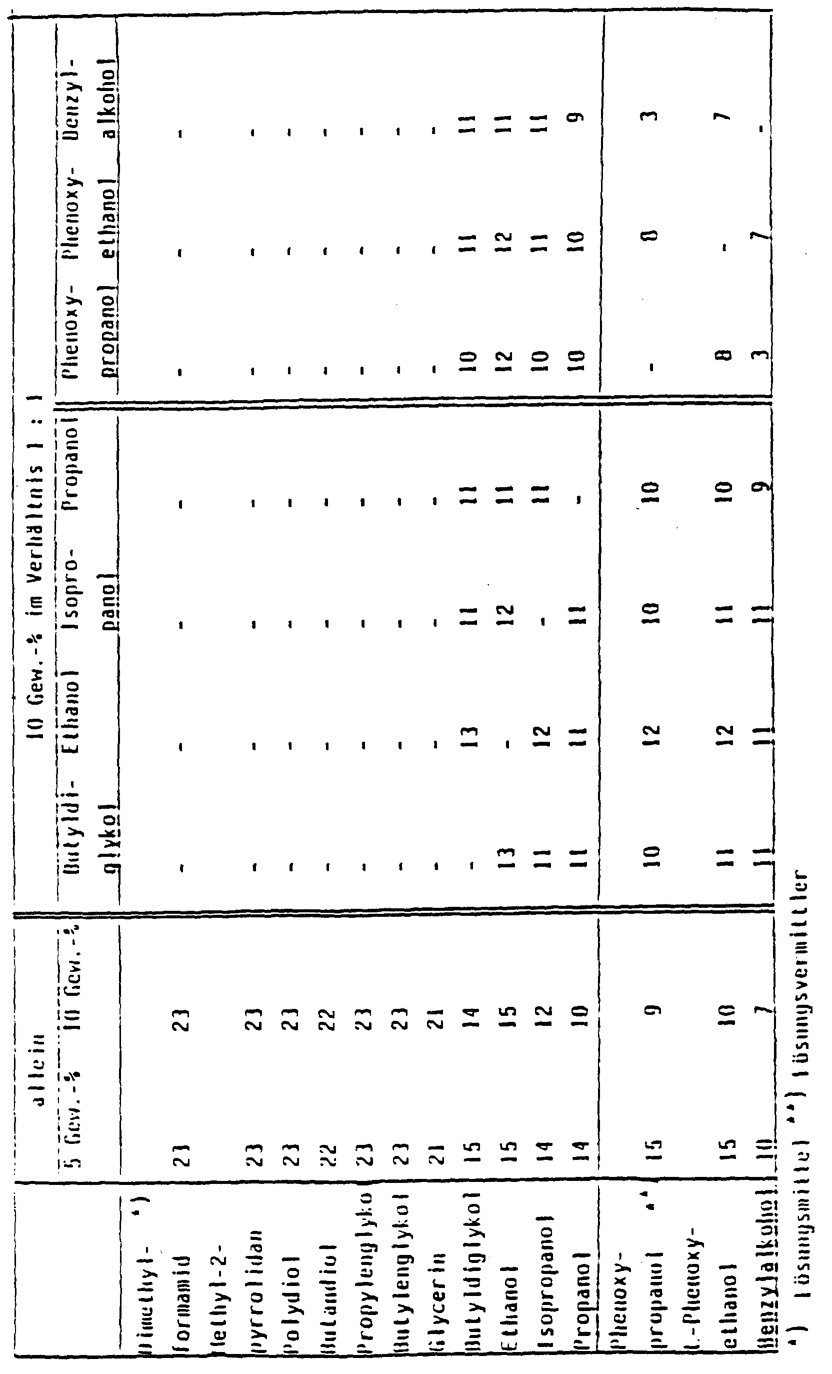

## Patentansprüche

1. Reinigendes wäßriges Desinfektionsmittel auf Basis von Umsetzungsprodukten aus
a) N-substituierten Propylendiaminen der Formel I:
R₁-NH-CH₂-CH₂-CH₂-NH₂, (I)
in der R₁ für einen linearen Alkylrest mit 12 bis 14 Kohlenstoffatomen steht, und
b) Verbindungen der Formel II:
R₂-O-CO-CH₂-CH₂-CH(NH₂)-COOH, (II)
in der R₂ ein Wasserstoffatom oder einen Alkylrest mit 1 bis 4 Kohlenstoffatomen bedeutet, wobei das molare Verhältnis von a : b 1 : 1 bis 1 : 2 beträgt, (Komposition U) und/oder deren Salzen mit anorganischen oder organischen Säuren, gegebenenfalls nichtionischen und/oder Amphotensiden, Komplexbildnern und organischen Lösungsmitteln gemäß EP 156 275, das dadurch gekennzeichnet ist, daß es zusätzlich als Lösungsvermittler Benzylalkohol und/oder 2-Phenoxyethanol und/oder 1-Phenoxypropanol enthält.

2. Mittel nach Anspruch 1, dadurch gekennzeichnet, daß es U in Mengen von 5 bis 40, vorzugsweise von 10 bis 25 Gew.-% enthält.

3. Mittel nach Anspruch 1 und 2, dadurch gekennzeichnet, daß es die Lösungsvermittler in Mengen von 3 bis 30, vorzugsweise von 7 bis 15 Gew.-% enthält.

4. Mittel nach Anspruch 1 bis 3, dadurch gekennzeichnet, daß das Gewichtsverhältnis von Lösungsvermittler zu U 1 : 6,0 bis 1 : 0,05, vorzugsweise 1 : 0,5 bis 1 : 0,07 beträgt.

5. Mittel nach Ansprucn 1 bis 4, dadurch gekennzeichnet, daß es als Lösungsvermittler ein Gemisch aus gleichen Gewichtsmengen an Benzylalkohol und 2-Phenoxyethanol oder 1-Phenoxypropanol enthält.

6. Mittel nach Anspruch 1 bis 5, dadurch gekennzeichnet, daß es im wesentlichen aus
10 - 25 Gew.-% Komposition U
10 - 20 Gew.-% Lösungsmitteln,
3 - 30 Gew.-% Lösungsvermittlern, wobei die Summe aus Lösungsmittel und Lösungsvermittler vorzugsweise etwa 35 Gew.-% nicht übersteigt,
3 - 20 Gew.-% nichtionischen Tensiden,
0 - 10 Gew.-% Amphotensiden,
0,5 - 3 Gew.-% Korrosionsinhibitoren,
0,2 - 1 Gew.-% Duft- und Farbstoffen und
Rest auf 100 Gew.-% Wasser
besteht.

## Claims

1. A cleaning, water-containing disinfectant based on a reaction product of
a) N-substituted propylenediamines corresponding to formula I:
R₁-NH-CH₂-CH₂-CH₂-NH₂ (I)
in which R₁ is a linear alkyl radical containing 12 to 14 carbon atoms,
and
b) compounds corresponding to formula II:
R₂-O-CO-CH₂-CH₂-CH (NH₂)-COOH (II)
in which R₂ is a hydrogen atom or an alkyl radical containing 1 to 4 carbon atoms,
the molar ratio of a) to b) being 1:1 to 1:2, (composition U) and/or salts thereof with inorganic or organic acids, optionally together with nonionic and/or amphoteric surfactants, complexing agents and organic solvents according to EP 156 275, characterized in that it additionally contains benzyl alcohol and/or 2-phenoxyethanol and/or 1-phenoxypropanol as solubilizer.

2. A disinfectant as claimed in claim 1, characterized in that it contains U in quantities of 5 to 40% by weight and preferably in quantities of 10 to 25% by weight.

3. A disinfectant as claimed in claims 1 and 2, characterized in that it contains the solubilizers in quantities of 3 to 30% by weight and preferably in quantities of 7 to 15% by weight.

4. A disinfectant as claimed in claims 1 to 3, characterized in that the ratio by weight of solubilizer to U is 1:6.0 to 1:0.05 and preferably 1:0.5 to 1:0.07.

5. A disinfectant as claimed in claims 1 to 4, characterized in that it contains a mixture of equal quantities by weight of benzyl alcohol and 2-phenoxyethanol or 1-phenoxypropanol as solubilizer.

6. A disinfectant as claimed in claims 1 to 5, characterized in that it consists essentially of
10 to 25% by weight of composition U,
10 to 20% by weight of solvents,
3 to 30% by weight of solubilizers, the sum of solvent and solubilizer preferably not exceeding about 35% by weight,
3 to 20% by weight of nonionic surfactants,
0 to 10% by weight of amphoteric surfactants,
0.5 to 3 % by weight of corrosion inhibitors,
0.2 to 1 % by weight of fragrances and dyes and
balance to 100% by weight water.

## Revendications

1. Agent de désinfection aqueux à action nettoyante à base de produits de réaction de
a) propylendiamines substituées par N de formule I,
R₁-NH-CH₂-CH₂-CH₂-NH₂ (I)
dans laquelle R₁ représente un radical alkyle linéaire ayant de 12 à 14 atomes de carbone, et,
b) des composés de la formule II,
R₂-O-CO-CH₂-CH₂-CH(NH₂)-COOH, (II)
dans laquelle R₂ représente un atome d'hydrogène ou un radical alkyle ayant de 1 à 4 atomes de carbone, le rapport molaire de a:b se montant de 1:1 à 1:2 (composition U) et/ou leurs sels avec des acides minéraux ou organiques, éventuellement ensemble avec des agents tensioactifs non ioniques et/ou ampholytes, des agents complexants et des solvants organiques selon le document EP 156 275, agent qui est caractérisé en ce que comme agent de solubilisation il contient de l'alcool benzylique et/ou du 2-phenoxyéthanol et/ou de 1-phenoxypropanol.

2. Agent selon la revendication 1,
caractérisé en ce que
il contient U en quantités de 5 à 40, de préférence de 10 à 25 % en poids.

3. Agent selon les revendications 1 et 2,
caractérisé en ce que
il contient les agents de solubilisation en quantités de 3 à 30, de préférence de 7 à 15 % en poids.

4. Agent selon les revendications 1 à 3,
caractérisé en ce que
le rapport de poids des agents de solubilisation à U se monte de 1:6,0 jusqu'à 1:0,05, de préférence de 1:0,5 à 1:0,07.

5. Agent selon les revendications 1 à 4,
caractérisé en ce que
il contient comme agents de solubilisation un mélange des mêmes quantités pondérales d'alcool benzylique et de 2-phénoxyéthanol ou de 1-phénoxypropanol.

6. Agent selon les revendication 1 à 5,
caractérisé en ce que
il se compose essentiellement de :
10-25 % en poids de composition U
10-20 % en poids de solvants
3-30 % en poids d'agent de solubilisation, tandis que la somme des solvants et agents de solubilisation ne dépasse pas de préférence environ 35 % en poids,
3-20 % en poids de tensioactifs non ioniques,
0-10 % en poids de tensioactifs ampholytes,
0,5-3 % en poids d'inhibiteur de corrosion,
0,2-1 % en poids de matières odoriférantes et colorantes et,
le reste à 100 % en poids d'eau.
